# EUROPEAN PATENT SPECIFICATION

(11) **EP 0 620 748 B1**
(45) Date of publication and mention of the grant of the patent: **28.07.2010**
(21) Application number: 93901055.9
(22) Date of filing: 05.01.1993
(51) Int. Cl.: A61M 5/20, A61M 5/32

(54) **AUTOMATIC INJECTORS**
AUTOMATISCHE INJEKTIONSGERÄTE
INJECTEURS AUTOMATIQUES

(30) Priority: 07.01.1992 GB 9200219; 26.08.1992 US 936236
(43) Date of publication of application: 26.10.1994
(73) Proprietor: Meridian Medical Technologies, Inc., Columbia, MD 21046 (US)
(72) Inventor: WILMOT, John, Glyndwr, Maryland 21771 (US)
(74) Representative: Lawrence, John
(86) International application number: PCT/GB1993/000006
(87) International publication number: WO 1993/013819

(56) References cited:
- WO-A1-88/00066
- DE-A1- 2 019 296
- FR-A2- 2 650 187
- GB-A- 728 248
- US-A- 2 674 246

## Description

This invention relates to automatic injectors of the kind comprising a body which contains a charge of medicament, a needle held in a sheathed position within the body, releasable drive means which when released drives the needle from its sheathed position to an unsheathed, projecting, position projecting from the body, and expulsion means for discharging the medicament through the needle. Automatic injectors of this kind will hereinafter be referred to as of the kind set forth.

Automatic injectors of the kind set forth have been developed primarily for use by persons who have to administer an injection into their own body at an instance which is not known beforehand. However, they may also be used by people who know that they will have to inject themselves, but are not skilled in the use of manually operated hypodermic syringes.

There are very many proposals for automatic injectors. Despite the existence of many different kinds of automatic injectors hitherto they may have been a menace after use. Furthermore, after use the user can see the needle projecting from the body of the injector.

German Specification 2 019 296 discloses a multi-use automatic injector that has an open-ended ring at its forward end which is advanced after injection to extend as far as the projecting needle. The ring can be readily retracted again and latched in its retracted position, the needle changed for a new needle, the drive spring re-compressed, and a new capsule of medicament introduced into the injector to ready the injector for another injection. The ring is not provided for "sharps" purposes, but in order to retract the needle from the body of the patient after injection.

The aim of the present invention, as described in claim 1, is to provide a new automatic injector which may be safer for use.

It solved the problem of the user or others accidentally pricking themselves by the features defined in the characterising part of claim 1.

More particularly, the needle is effectively only out of the injector whilst it is in the user's body during the injection operation. This has the further advantage that the needle is not seen by the user either before or after the injection operation.

The cover member may have a pre-injection position relative to the body and be movable rearwards relative to the body to the injection position. The pre-injection and post-injection positions of the cover member relative to the body may be substantially the same, at least longitudinally. Alternatively the pre-injection position of the cover member relative to the body may be further rearwards longitudinally of the injector than the position of the cover member in its post-injection position. This enables the unactuated injector to be more compact longitudinally than it would be if the pre- and post-injection positions were the same longitudinally. The pre-injection position may be about half, two-third, or four fifths of the way from the injection position of the cover member towards the post-injection position of the lower member.

The cover member may be moved to its injection position by the action of pressing the injector against the person of the user prior to activating the injector.

Preferably the cover member comprises a sheath, cap or tube, preferably retained to the body and axially movable relative thereto. The sheath is preferably movable between two axially spaced stops.

The cover member may have or be engaged by a manually releasable detent the release of which enables the sharps assembly to operate. The detent may comprise a tear-off tab, band or the like.

Preferably the cover member is lockable in its post-injection position. Alternatively the cover member may have a locked position which is different from the, unlocked, post-injection position, the needle still being protected in the locked position.

The cover member may be automatically locked after injection, or it may be manually lockable. The cover member may be locked by moving it axially or angularly, or a combination of both, relative to the injector body. For example, it could be locked following injection by moving it from its initial post-injection position further away from the body of the injector, or by turning it relative to the body. The locking mechanism could conveniently be one-way snap-engagement features, or a non-returnable ramp or cam system.

In the case of automatic operation of the sharps assembly and automatic locking of the cover in its post-injection position the user has substantially no opportunity of accidentally pricking anyone since the needle is effectively only out of the injector whilst it is in the user's body. This has a further advantage in that the needle can never be seen.

The injector may have locking means for the cover member comprising a snap-fit lock. The locking means may comprise a bayonet formation on the cover member or body.

Locking of the cover member may be effected by relative angular and/or axial movement between the cover member and the body.

Mis-alignment means may be provided between the cover member and the body to mis-align a needle aperture or weak area provided in the cover member with the (projected) needle after the injector has been fired, thereby preventing accidental relative movement of the cover member towards the body which could otherwise enable the projected needle to protrude beyond the cover member.

The mis-alignment means may comprise cocking, twisting, or tilting means adapted to tilt the cover member relative to the body to a non-coaxial configuration.

The mis-alignment means may mis-align the needle aperture, or weak area, in the cover member relative to the needle in the body when the injector is in its pre-firing, unoperated, state.

Embodiments will now be described by way of example only with reference to the accompanying drawings of which:-
Figures 1 to 3 show schematically a first automatic injector in configurations, respectively, before use, during use, and after use;
Figure 4 shows detail of the automatic injector of Figures 1 to 3;
Figures 5 to 7 show schematically a second automatic injector in configurations, respectively, before use, during use, and after use;
Figures 8 to 10 show schematically details of a safety assembly of a third automatic injector in configurations, respectively, before use, during use, and after use;
Figure 11 to 13 show schematically details of a safety assembly of a forth automatic injector in configurations, respectively, before use, during use, and after use;
Figures 14 to 16 show schematically a fifth automatic injector in configurations, respectively before use, during use, and after use;
Figure 17 shows a modification of, the injector of Figures 14 to 16 according to the invention;
Figures 18 shows a section on the curved line from point A to point B of the modification of Figure 17;
Figure 19 shows a section on the curved line from point C to point D of the modification of Figure 18; and
Figures 20 to 22 show schematically a further modification of an injector according to the invention in configurations, respectively before use, during use, and after use;
Figures 23 shows yet another modification of an injector;
Figures 24 shows a modification of the injector of Figures 8 to 10;
Figures 25 shows detail of another injector;
Figures 26 shows a modification of the injector of Figures 17 to 19 according to the invention;
Figures 27A and 27B show cross-sections on curved lines A-A and B-B of Figure 26; and
Figures 28A to 28C show schematically the forward end of the injector of Figure 26 in its pre-injection, during-injection, and post-injection positions respectively.

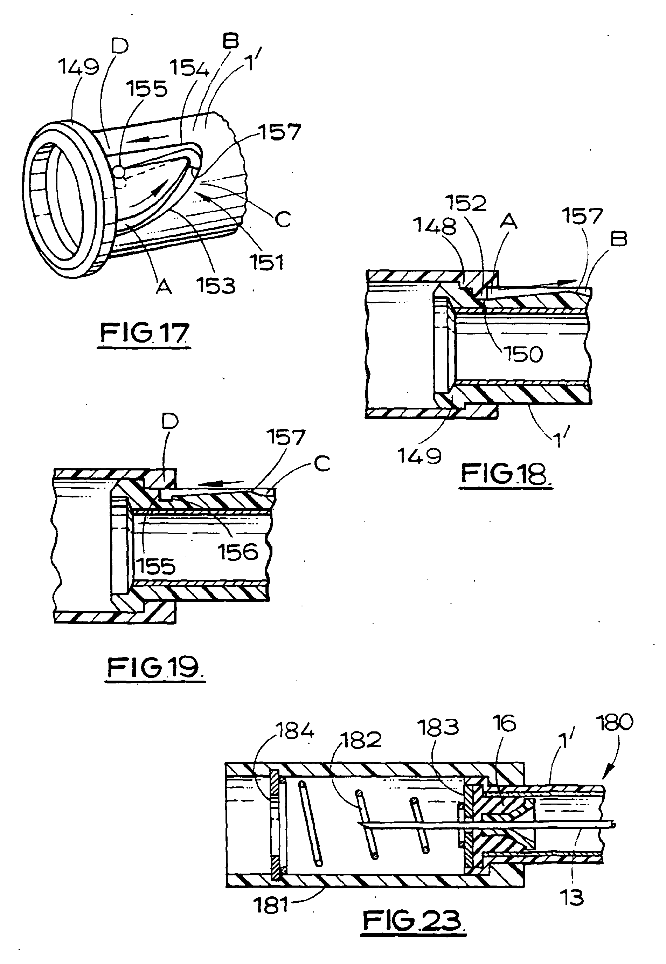

Only the embodiments of figs. 17-22 and 26-28 come within the scope of the present invention.

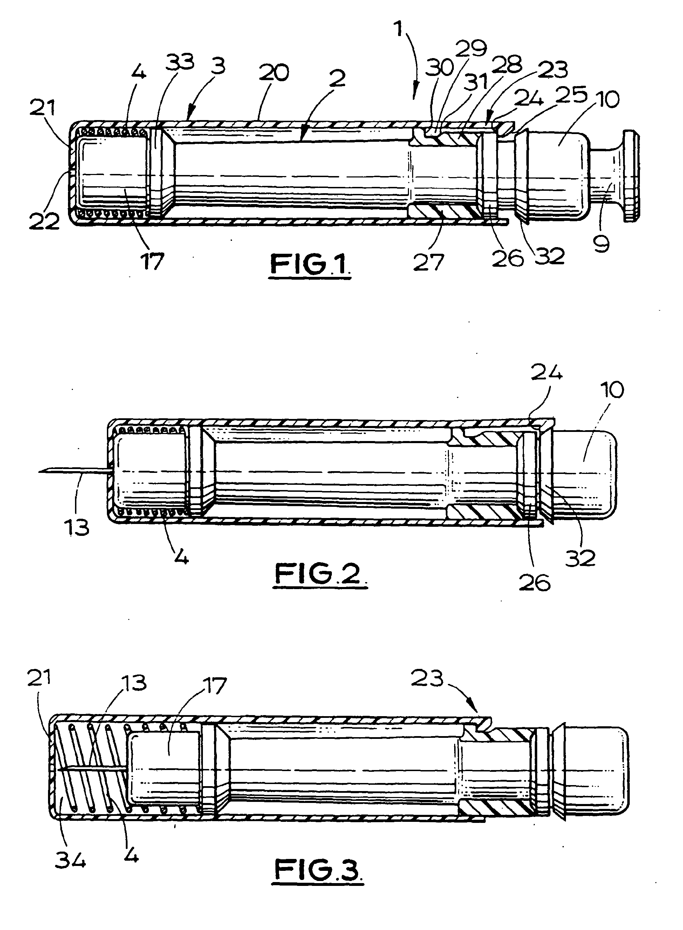

An automatic injector 1 is shown in Figures 1 to 4 which comprises an injector assembly 2, a plastics protective, sharps, cover member 3, and a sharps spring 4.

The injector assembly 2 is of a known general kind, and the particular details of its structure are not of prime importance to the broadest aspects of the invention. In the example of Figures 1 to 4 the structure of the injector assembly is generally the same as that described in our published European Patent Application No. 0567483, but it will be appreciated that many other injector assembly arrangements can be used instead.

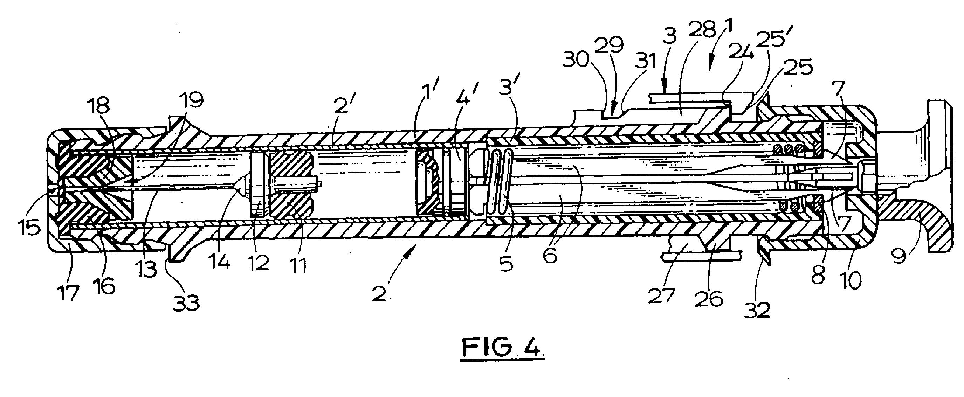

Figure 4 shows details of the injector assembly 2 which comprises a body 1' of injection-moulded polystyrene containing a barrel liner 2' of F.E.P. 160 and a spring casing 3' of polystyrene. Sliding within the barrel liner 2' is a first piston 4' of rubber acted on by a stainless-steel coil compression spring 5. In the initial condition of the injector the spring is held in the compressed position, as shown in the drawing, by a collet 6 made in two halves having at their tail ends detent teeth 7 engaging a latch ring 8 seated in the end of the spring casing 3'. A safety pin 9 of moulded nylon normally keeps the teeth 7 apart but when it is withdrawn they can be urged together to release the collet 6 by a short movement of an end cap 10.

This spring-restraining and release mechanism is know and is substantially the same as that disclosed in our above-mentioned Patent specification.

Also within the barrel liner 2' and spaced about half way along in the initial condition is a second piston 11, also of rubber. Sealed into this piston is a moulded polyethylene needle-mounting 12 carrying the injection needle 13 sealed into it by adhesive 14.

In the condition shown, the tip of the needle 13 stops just short of a diaphragm seal 15 formed in a bush 16 which is held in the end of the barrel liner 2' by an end cap 17.

The tip of the needle 13 is received in a guide 18 of HD polyethylene shaped as shown, with its outside fitting into the bush 16 and its inside a good sliding fit on the needle. At its inner end the guide has a convergent conical portion 19 which helps to lead the needle into the bore of the guide during assembly of the injector.

The space between the pistons contains the medicament and is in communication with the open rear end of the needle. The space between the second piston and the end of the body, i.e. the space around the needle, contains air or an inert gas.

When the injector is put to use by removing the safety pin 9 and actuating the end cap 10 to releases the collet 6, the spring 5 initially advances both pistons together, as the liquid between them is virtually incompressible. The needle 13 advances through the guide 18 and penetrates the seal 15, emerging through the centre of the hole in the end cap 17. The air or gas in the space around the needle is able to force its way between the outside of the needle and the bore of the guide 18, and so does not hold up the advance of the second piston. This is an important feature of the injector assembly 2 (but not necessarily of the present invention) and it means that the needle is able to advance fully into the patient's body until brought to halt when the mounting 12 comes up against the guide 18, and thereafter medicament is injected by the continued advance of the first piston. Only a negligible quantity, if indeed any at all, is injected during the advance of the needle.

The cover member 3 substantially surrounds the body 1' and has side walls 20 of larger internal diameter than the outer diameter of the body 1, a front wall 21 having a central hole 22, wand carries one or more resilient deflectable fingers 23 at its rear end. The finger 23 has a latching face 24 and a chamfered face 25.

The body 1' of the injector assembly 1 has at its rearward end a radially projecting latching rib 26 and one or more guide fins 27,28 extending radially outwards adjacent the rib 26. At least one of the fins, fin 28, has a recess 29 axially spaced from the rib 26 and defines forward and rear abutment faces 30 and 31. The rearward end cap 10 has a chamfered end face 32. The forward end of the body is provided with an abutment rib 33 adjacent the end cap 17. The spring 4 surrounds the end cap 17 and abuts against the rib 33, continually urging the front wall 21 of the cover member 3 away from the body 1.

In the pre-injection condition shown in Figure 1 the finger 23 is latched onto the rib 26 and the cover member 3 is held in place against the end cap 17, with the spring 4 compressed.

When the user wishes to use the injector he places the front wall 21 against his leg (or other body part, or against the body of someone else to be injected), then removes the safety pin 9 and presses the end cap 10. The face 32 engages the face 25 and biases the finger 23 radially outwards, releasing the finger from its engagement with the rib 26. Simultaneously the spring 5 is released and the needle 13 is driven through the hole 22 and into the user. The hole 22 may be covered by a membrane for hygiene purposes.

Since the end face 32 of the cap 10 is now adjacent the rib 26, as shown in Figure 2; when the user moves the injector away from his leg the head of the finger 23 cannon return to its radially inward position of Figure 1 (there is not a big enough gap between the rib. 26 and the end cap 10) and the spring 4 moves the cover member forward to its advanced positions shown in Figure 3 in which the needle 13 is held in a chamber 34 defined by the cover member. The head of the finger 23 is received recess 29 and the face 24 of the finger 23 is engaged against face 30 and this restrains further forward movement of the cover member.

In the embodiment of Figures 1 to 4 the cover member 3 is advanced automatically, but it may possibly be moved manually back against the action of spring 4 because of the possibility of co-operation between the sloping faces 25 and 31.

In a modification the face 31 is not sloped, instead it forms an abutment preventing the return of the finger in an axial direction. For example the flat end face of the finger, referenced as 25' in Figure 4, may abut a complementary radial face. Thus the sharps system is locked in its needle-covering position.

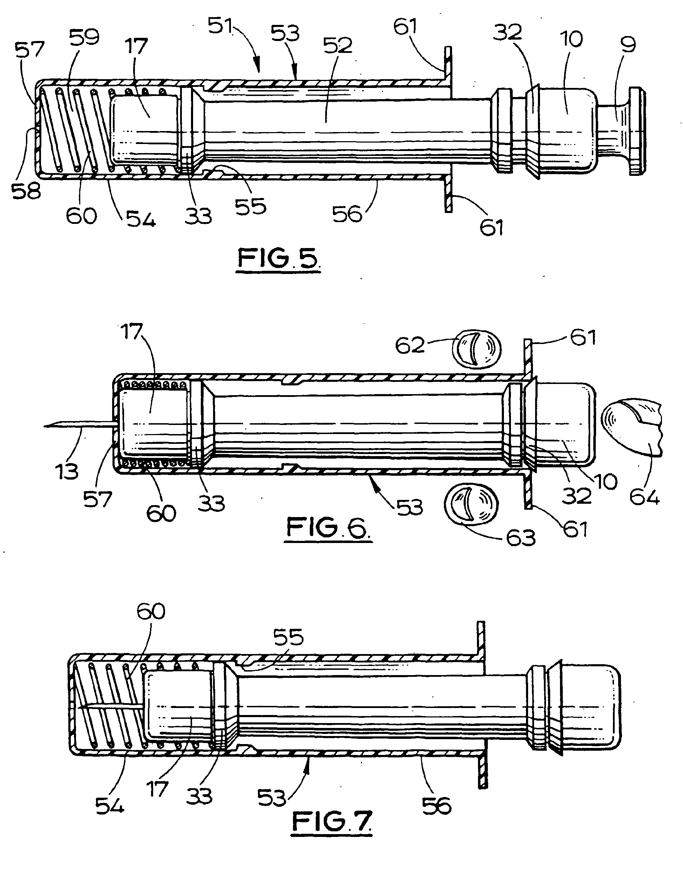

Figures 5 to 7 show another automatic injector 51 incorporating an automatic sharps system. The injector 51 comprises an injector assembly 52 which is similar to the assembly 2 of Figure 4, (and similar components have been given the same reference numerals) and a generally cylindrical cover member 53. The cover member 53 has a forward portion 54, an internal annular abutment ridge 55, and a rearward portion 56.

The forward portion 54 has a front wall 57 provided with a central hole 58 and in the pre-injection condition shown in Figure, 5 defines a space 59 between the forward end cap 17 of the assembly 52 and the front wall 57. A spring 60 engages the wall 57 and the abutment rib 33 of the injector assembly 52 and continually urges the wall 57 away from the rib 33.

The rearward portion 56 of the cover member 53 has a pair of radial arms 61 extending away from it at its rearmost end, which is open. The injector assembly 52 is received in the cover member 53 and is substantially surrounded by it, the end cap 17 of the injector assembly 52 being held in the space 59 by the ridge 55.

To use the injector 51 the user places the front wall 57 of the member 53 against his, or someone else's, body part, removes the safety pin 9, places his index finger 62 and middle finger 63 in front of the arms 61, and his thumb 64 on the rear end cap 10, and then presses the end cap 10 with his thumb, in the manner of operating a manual hypodermic syringe.

The initial phase of pressing of the cap 10 compresses the spring 60 until the front end cap 17 engages the front wall 57. The second phase of pressing the end cap 10 releases the detent teethe 7 and spring 5 of the injector assembly 52, thereby firing the needle 13 into the user's body and injecting the contents of the injector. The strength of the spring 60 and the force necessary to release the detents 7 are arranged appropriately for the spring 60 to be compressed before the spring 5 is released.

When the user removes the injector for disposal the spring 60 moves the cover member 53 over the needle and surrounds the needle with the rigid forward portion 54.

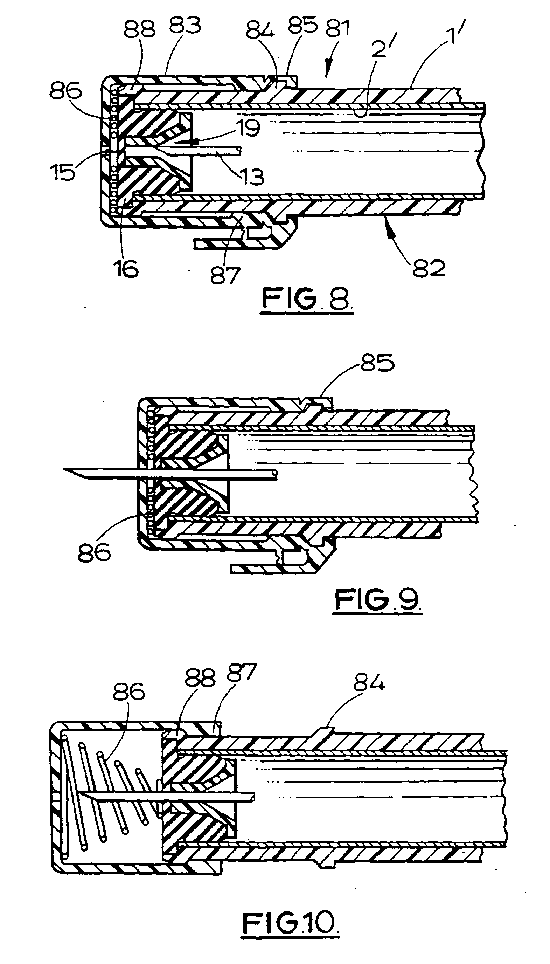

Figures 8 to 10 show a modified injector 81 having an injector assembly 82 and a cover member 83 attached to the end of a body 1' of the assembly 82. The assembly 82 is very similar to that of Figures 1 to 4 and the same reference numerals have been given to similar components.

The body 1' of the assembly 82 has an eternal flange 84 which co-operates with a tear-off strip 85 of the cover member 83. The cover member 83 can be considered to be similar to end cap 17 of the arrangement of Figure 4, except that it is biased by a spring 86 away from the bush 16. The spring 86 engages the end of the body 1' and the inside of the front wall of the cover member 83.

The injector 81 is used in the same way as the injector of European Patent Application No. 0567483, except that, after injection; the user tears off strip 85 which releases the spring 86 and causes the cover member 83 to move forward and enclose the projected needle. The cover member 83 and body 1' have complementary lugs 87 and 88 which prevent relative longitudinal movement beyond the position shown in Figure 10.

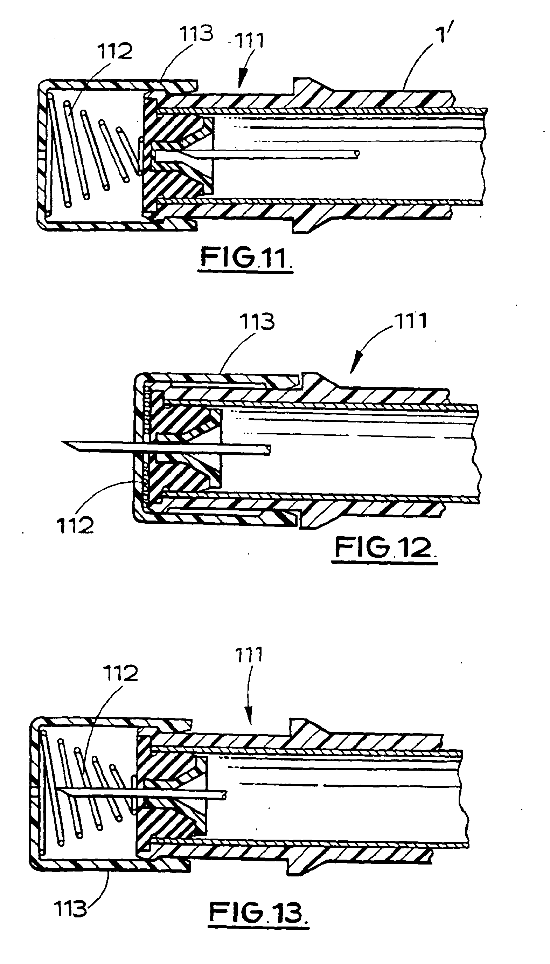

Alternatively the strip 85 could be torn off before use, which would give an injector similar to the injector 111 illustrated in Figures 11 to 13. The injector 111 has a spring 112 and a cover member 113.

Injector 111 can be arranged to operate in a similar manner to injector 51 in that its spring 112 is compressed by the action of pressing on its rear end cap, actuation of the injector occurring after the injector assembly has moved towards the cover member. Alternatively, the user may push the cover member 113 of the injector 111 against his person by holding the sides of the body 1' and then actuate the injector. This could, of course, also apply to the injector 51.

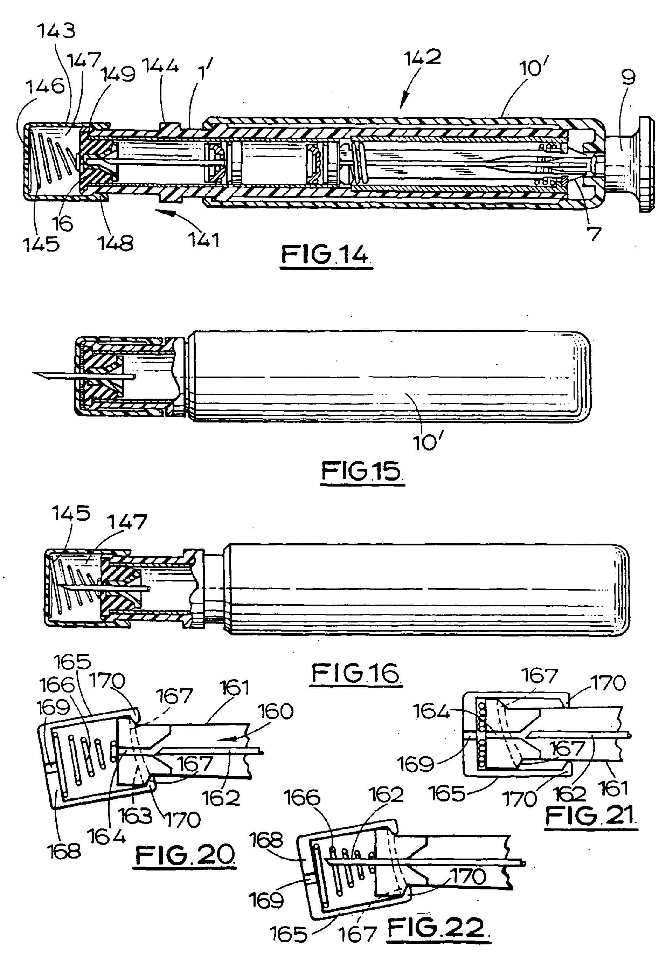

Figures 14 to 16 show an injector 141 having an injector assembly 142 and a cover cap or member 143. The injector assembly is largely as shown in our published European Patent Application No.0 361 668.

The assembly 142 comprises many components similar to those of assembly 2 and similar components have been given the same reference numerals.

The body 1' of the injector assembly 142 has towards its forward end an abutment flange 144. The rear end cap, referenced as 10', is longitudinally elongated and extends from the rear end of the body 1' to just before the flange 144 (in the pre-injection position shown in Figure 14). A spring 145 bears against a front wall 146 of the cover member 143, and against the bush 16, and continually biases the cover member 143 away from the body 1' so as to define an enclosure 147. The cover member 143 has an annular projection 148 at its rear end which co-operates with a complementary projection 149 on the end of the body 1' to retain the member 143 to the body.

The user places the front wall 146 against the area to be injected, removes the safety pin 9 and presses on the rearmost end of the end cap 10'. The spring 145 is compressed until the end of the cylindrical side walls of the cover member 143 engage the flange 144 whereafter force is transferred back to the detents 7. Further pushing on the end cap 10' causes the detents 7 to be released, the needle to be injected into the recipient, and the contents of the injector 141 to be injected. After removal of the injector from the user's body the spring 145 moves the cover member 143 over the needle to hide it.

Any or all of the infectors described could have a locking arrangement for their cover members such that once the cover member moves so as to hide the needle after injection it can be manually, or automatically, locked against retraction such as would expose the needle again.

A locking condition of the cover member may be achieved in substantially the post-injection positions shown in the drawings, or there may be further relative movement between the cover member and the body to a locked position.

One possible automatic locking mechanism is shown in Figures 17 to 19. For the sake of convenience the arrangement will be described with reference to the embodiment of Figures 14 to 16, but the structure is of course applicable to any of the injectors.

The cover member 143 has means, bayonet means 150, which co-operates with means, track or groove means 151, provided at the end region of the body 1' such as to provide a one-way path to a locking position as the cover member 143 is moved towards the body 1' and then released away from it again.

The bayonet means 150 comprises a stud 152 projecting radially inwards from the ring of the annular projection 148. The cover member 143 has a longitudinal slot provided in it adjacent the stud 152 so as to enable the side wall of the member 143 which carries the stud 152 to flex radially outwards, as will be described.

The track means 151 comprises a portion 153 extending rearwards, and a portion 154 extending back forwards. A locking hole 155 is provided at the forward end of the portion 154. The locking hole 155 has a step 156. The rearwardly extending portion 154 of the track 151 has a sloping base which is deeper at its forward end, and reaches its shallowest point (referenced as 157) before its rearmost point, and then becomes deeper again. This is the portion A to B shown in Figures 17 and 18.

The forwardly extending portion 154 of the track 151 has a base which slopes from a shallow end (point B) spaced from the forward end of the body 1' to a deeper end (point D) adjacent the forward end of the body. The locking hole 155 is at the deeper end of the forwardly extending portion 154. The rearwardly extending portion 153 is curved and bends to meet the forwardly extending portion 154. of course, the track 151 could have regions where its base is flat.

In use, as the cover member 143. is moved towards the body 1' the stud 152 rides up the slope of the rearwardly extending portion 153 of the track until it reaches point 157, the side wall of the cover member flexing radially outwards, and then rides down the slope beyond point 157 and around the bend in the rearwardly extending path 153. As the cover member moves forwards relative to the body 1' the stud 152 has a tendency to move "downhill", which guides it to move along the portion 154 of the path, rather than returning along portion 153. The stud 152 snaps into hole 155 and the step 156 prevents the cover member from being pushed back again relative to the body 1'.

It will be appreciated that in the embodiments of Figures 1 to 4, and 8 to 10, where the cover member doers not move towards the body before being advanced, there is no need for such a complicated one way track. The locking means could simply comprise the forward portion of the track.

Of course, the stud could be on the body and the track in the cover member. There may be more than one stud and associated track. The forwardly extending portion of the track may be curved, either in addition to or instead of the rearwardly extending portion. (when provided)

Figures 20 to 22 show another modification which is applicable to any of the injectors described. Figure 20 shows an automatic injector 160 before use. The injector 160 has a body 161, a needle 162, a needle guide 163 having a needle hole (or thin-walled region) 164, a cover member 165, and a spring 166. The front end of the body 161 has a flange or shoulder 167 which is inclined relative to a plane normal to the needle 162. The cover member 165 has a front wall 168 having a through-hole (or a thin walled region) 169 through which the needle 162 extends in use, and a rearward flange 170 which co-operates with a flange 167 of the body. There may be a membrane (which may be similar to seal 15 of the arrangement of Figure 4) provided at the forward end of the injector, the needle piercing the membrane during injection.

The spring 166 urges the front wall 168 away from the body 161, with the flange 170 engaging against the rearmost portion of the flange 167. Since the flange 167 is angled relative to the body (it is not perpendicular to it) the cover member 165 is urged by the spring 166 to a cocked position, shown in Figure 20, until the flanges 170 and 167 engage at both side of the body (at the top and bottom as seen in 20 to 22). The hole 169 is mis-aligned with the hole 164.

When the user presses the front wall 168 against his leg the cover member 165 is moved towards the body 161 and the cover member and body take up a concentric configuration, with the holes 164 and 169 aligned as shown in Figure 21.

When the automatic injector operates the needle passes through the holes 164 and 169 into the user's body (and through any sealing membrane which may be provided).

When the user removes the used injector from his body part that has just been injected the spring 166 pushes the cover member 165 to its advanced position. As the cover member slides forwards the needle 162, which is projecting from the body, keeps it concentric with the body until the front wall 168 moves beyond the forwardmost tip of the need whereafter the spring 166 again causes the cover member to take up its cocked configuration because of the inclined flange 167. Accidentally pushing on the front wall 168 of the cover member now causes the front wall 168 to engage the needle and this stops the cover member from being moved further towards the body and ensures that the needle is sanely covered. Accidental re-location of the needle 162 in the hole 169 is very unlikely and the mis-alignment of the needle and hole 169 effectively serves as a lock for the cover member.

Of course, the flange 170 could be inclined so as to serve as mis-alignment means, either in addition to, or instead of, the inclining of flange 167.

The forward end portion of a modified injector 180 is shown in Figure 23. The injector 180 has two modifications of note, the first of which is that instead of having an end cap with a small needle aperture to define a sharps enclosure for the needle, it has an open-ended sheath, sleeve, or tube, 181. This may be easier or cheaper to manufacture. The protective sheath 181 may need to project further than an equivalent end cap with a substantially closed end in order to protect the needle properly. It may even be acceptable to allow a user deliberately to insert his finger into the open end of the sheath and reach the needle, so long as it is unlikely to occur accidentally. On the other hand the sheath 181 may be too narrow or too long (in its advanced position) to allow an adult, and/or a child, to push their finger inside it and reach the needle after firing of the injector.

The injector 180 also has a second difference from those previously described and that is that the rear end of the sharps spring 182 (which urges the sheath forwards) bears against a plastics or metal load-spreader plate 183 which in turn bears on the rubber bush 16. This spreads the load of the spring and avoids the bush 16 deforming locally as it might if the spring 182 were to contact it directly (and over a smaller area). The load spreader plate 183 may engage a radial shoulder on the forward end of the body 1', thereby transferring force directly to the body, without stressing the bush 16 significantly. Alternatively, another way of avoiding applying excessive load to the rubber bush 16 is to have the plate 183 integral with the body 1' of the injector. This is effectively mounting the spring 182 on an end cap of the injector.

In the arrangement of Figure 23 the spring 182 bears against a circlip 184 received in a groove in the inner wall of the sheath 181. The circlip 184 and/or spring 182 may serve as an obstacle to the user's finger if the.user tries to reach the needle.

It will of course be appreciated that the feature of an open-ended sheath providing the cover member for the needle, and the feature of the spring bearing on a load-spreader plate, or an end cap of the injector, can apply to any of the embodiments described.

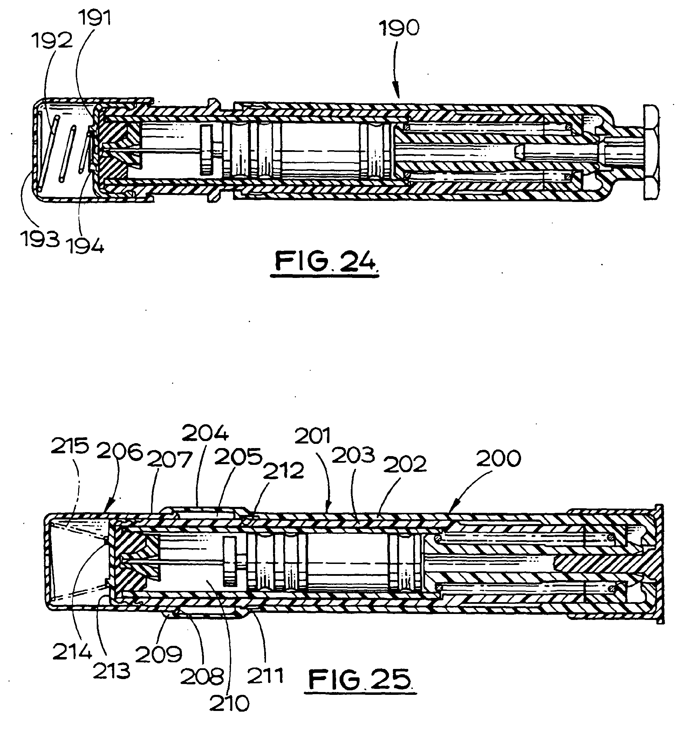

Figure 24 shows an arrangement similar to the arrangement of Figures 8 to 10, but modified along the lines mention above. The injector, referenced 190, has a rigid plastics material end cap 191 similar to that disclosed in our co-pending patents already referred to, and a spring 192 urging a sharps cover 193 away from the end cap 191. The end cap has a centering, or retaining, ring 194 moulded on its forward face which serves to locate one end of the spring 192. The end cap 191 is permanently fixed to the body of the injector.

Figure 25 shows another injector 200. The injector has a body 201 formed from an outer tubular member 202 and an inner tubular member 203. The outer member 202 is provided with a collar 204 which defines in part an annular space 205. The inner member 203 also helps to define the space 205. A sharps cover 206 is provided extending over the forward end of the inner member 203. The cover 206 has sidewalls 207 which are capable of entering the space 205, and retaining formations 208 which are trapped in the space 205 by a shoulder 209 provided on the collar 204. The collar 204 is in this example provided on the outer tubular member 202 and the separate inner member 203 which contains a medicament chamber 210. The outer member 202 has an annular groove 211 which receives projecting locating formations 212 of the inner member 203 in a snap-fit manner. The outer member 202 can move slightly axially relative to the inner member 203 to actuate the injector. The injector has an end cap 213 which has a locating formation 214 to locate a spring 215 which bears against the cover 206.

In use the rearward part of the side walls of the cover slide into the internal space 205. Because the cover 206 slides in an internal space the space cannot easily become blocked with dirt or other obstructions.

The arrangement of Figure 25 is front actuated in that the user must press the front of the injector against his body for the injector to operate. The cover 206 must therefore be in its retracted position for the injector to operate. It is not possible for the user to actuate the injector without retracting the cover 206. This avoids any possibility of the user injecting into the space defined by the cover member when it is in its advanced position. Similarly it is very difficult for a user to actuate the injector of Figure 24 without compressing the cover member. The only way he can do it is to grip the body of the injector just behind the cover member 193. This is unlikely to occur accidentally. Furthermore, the injector of Figure 24, and indeed any of the automatic injectors, can be made front actuated fool-proof injectors by using the principle of an outer sleeve extending substantially the full length of the injector as exemplified in Figure 25.

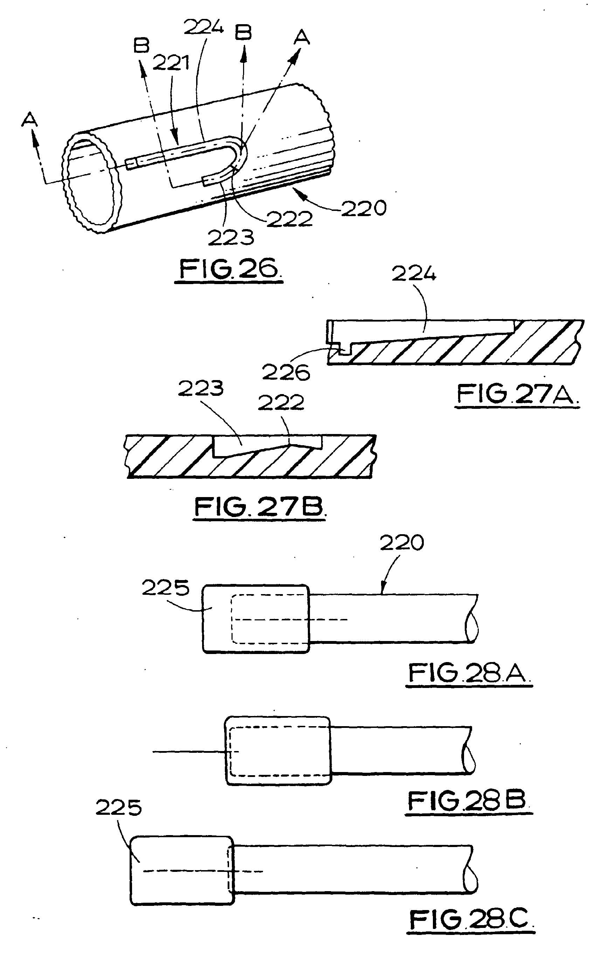

Figures 26 to 28C illustrate a way of reducing the axial length of an injector having a sharps system. It is desirable to have an injector in accordance with the invention, but which is not too much longer than a comparable injector which does not have the sharps system. The injector, referenced as 220, has a guide track 221 comparable with track means 151 of the embodiment of Figures 14 to 16. However, instead of the track 221 having rearwardly and forwardly extending portions, 223 and 224 respectively, of equal longitudinal, axial, length, the forward portion 223 takes the cover member (referenced as 225 in Figures 28A to 28C) further forwards than it is in its pre-injection position. The track has a double cam, with crest 222 being shown. The cover member can be considered to be about 80% retracted towards its injection position (20% advanced towards its post-injection position) in its pre-injection position.

There is a snap-fit lock 226 (see Figure 27A) operable at the post-injection position to prevent the cover member from being retracted again. The cover member 225 has a pin, or the like, which is guided in the track 221, and locking means (usually the guide pin) which co-operates with the snap-fit lock 226.

There may also be a lock, or releasable latch means, to hold the cover in its pre-injection condition against accidental movement therefrom. The pre-injection latch could comprise a component which is manually destroyed before the injector is used (such as a tear-off band), or it could comprise a snap-fit latch, for example a stud engaged in a complementary hole.

It will be appreciated that it is preferable to have the spring which engages the sharps cover member weaker than that the force necessary to be applied to the rearward end cap of the injector to actuate it. This means that when the injector is placed against a user and its rearward end cap is pressed to actuate the injector, the injector will fire only after the sharps cover member has been fully retracted.

The sharps cover member may be manufactured by moulding it as a cylinder. Alternatively we may find it better to mould it as two half-cylinders, possibly joined by a living hinge, and to join the two half cylinders together around the forward end of the body of the injector to assemble the sharps cover member, and simultaneously attach it to the body of the injector. The two half-cylindrical portions of the sharps cover member may have complementary interengagable snap-fit formations to enable the two halves to be joined together with a snap-fit operation.

We may also care to mould the sharps spring into the sharps cover member. There may be a plastics moulding spring.

Another arrangement for a fully automatic sharps system for an automatic injector comprises having a resilient compressible member, such as a foam sponge or the like, mounted at the front end of the injector (for example bonded to the front end cap, such as cap 17 of Figure 4). When the injector is unactuated its front end (i.e. the resilient compressible member) can be pressed against the user's body prior to release of the drive means, compressing the axial length of the resilient compressible member, and thereby bringing the user's body part within range of the needle when the injector is actuated. Upon actuation the needle passes through the resilient compressible member, which may have a hole to assist this, and into the user's body part..As the user moves the injector away from his body after injection the resilient compressible member expands again to cover the needle. Thus after use the needle is encased in the resilient compressible member, giving sharps protection - or at least a degree of protection. The needle is hidden by the resilient compressible member and can never be seen.

Instead of having a resilient compressible member which is compressed in use and then expands, the injector could be provided with an expandable member which is in a compressed condition, or a partially compressed condition, during storage of the injector (and during an injection) and which is released to expand and cover the needle after injection. The expandable member preferably expands automatically after injection to provide an automatic sharps system. The expandable member may comprise a resilient material, such as a foam sponge.

The resilient compressible member or expandable member could be provided as the cover member, or inside or behind a more rigid cover member (thereby acting as a spring).

A further modification may be to have the resiliently compressible member, or expandable member, comprise a helical spring. The coils of the spring can take the place of side walls of a rigid cover member, the released spring surrounding the needle and thereby comprising a cover member. The spring may have an open end (akin to the arrangement of Figure 23) or it may have an end plate with a needle aperture. Even though a tubular spring surrounding a projecting needle is not rigid it can still be an effective cover member, for example the end plate could have its needle aperture mis-aligned with the needle after injection. The spring could have a bent, non-straight, relaxed condition. The spring could be a conical spring so that the projected needle cannot be seen between the coils of the expanded spring, or at least is substantially obscured from view.

The sharps assembly, of whatever kind, may be released for movement by the action of injecting. For example it may be released (as in the arrangement of Figure 26) by the rearward movement of the cover member. Alternatively the sharps assembly may have release means actuated by movement of the drive means or expulsion means.

In yet another embodiment of the invention an automatic injector may have a cover member screw-threaded onto the forward end of the body of the injector. Automatic unscrewing means (such as a spiral/torque spring) is provided acting between the cover member and the body. The cover member has a release feature akin to that of Figures 17 and 26 in that the cover member is initially in an intermediate axial position, pressing the cover member against the user's body moving the cover member back a distance (say 20% of its eventual forward travel). This rearward movement of the cover member releases the screw-action locking mechanism - (it may allow the screw thread on the cover member to engage the rear portion of the screw thread on the body, or they may already be interengaged). After the injector has been fired it is removed from the skin. The automatic unscrewing means operates to unscrew the cover member thereby extending the cover member forwards. The screw threads have a sufficiently low helix angle that when the cover member is extended merely pressing it back axially has little effect: an angular torque is required for ease of movement. This is less likely to happen accidentally.

The automatic unscrewing means may advance the cover to such an extent that it is fully unscrewed from the thread on the body - the screw threads may no longer co-operate. The cover member is then, of course, still retained to the body by retaining means to stop it falling off. Since the screw threads of the cover member and body do not now mate the cover member cannot be accidentally pushed back to reveal the needle. Thus the screw threads provide automatic locking of the cover member in a needle-protecting position (even though the cover member may not be rigidly held to the body it is still prevented from exposing the needle accidentally).

Any of the features of any one of the injectors disclosed may be used in combination with another injector.

Tear-off bands are considered advantageous because they can be used to keep an injector in a retracted, compressed, condition prior to use, and so keep the part of the injector against which the cover member slides covered up so that it cannot become clogged up with dirt or other obstructions. A tear-off band could be used even when the injector has its cover member in an advanced, or partially advanced, position when the injector is in its storage condition simply to ensure that the area of the injector body that is to have the cover sliding back over it in use is not liable to become blocked. The tear-off band could be removed immediately prior to using the injector. Preferably removal of the band enable the injector to be actuated. A tear-off band, or some other lock, is very useful on automatic injectors which have sharps systems which lock in place when fully advanced in order to prevent accidental movement of the sharps cover to the advanced position before the injector is used (thereby locking the cover in its advanced position and making the unactuated injector unusable).

A particular advantage of an automatic injector which has an automatic sharps system is that the needle is never seen by the user. The only time that the needle protrudes from the injector is when it is in the user's body. As discussed, this improves the safety of the system. A further advantage is that some people are afraid of needles and it can be comforting not to have to see them. This can allow some people to use injectors who would otherwise be psychologically unable to use them.

One of the areas where we see our automatic injectors having an automatic sharps system being used is to combat male impotence. A man can simply place an automatic injector at the base of his penis and inject himself without needing any particular skill and he need never see the needle. This can readily be appreciated as being an advantage for this particular use, given the natural fear of placing exposed sharp objects near the genitals.

The invention can also be used with automatic injector systems which have a pre-filled syringe which is loaded into a re-usable or single use firing mechanism.

The embodiments shown in Figures 1 to 4; Figures 5 to 7; Figures 8 to 10; Figures 11 to 13; Figures 14 to 16; Figure 23; Figure 24 and Figure 25 of the drawings fall outside the scope of the claims and are given for example only.

## Claims

1. An automatic injector (1;141;160;220) comprising a body (11;161), a charge of medicament contained in the body (11;161), a needle (13;162) initially held in a sheathed position within the body (11;161), releasable drive means (5,6) for driving the needle (13;162) from the sheathed position to an unsheathed position in which the needle (13;162) projects from the body (11;161), the releasable drive means (5,6) further forcing the medicament through the needle (13;162), a protective assembly for covering the needle (13;162) in the unsheathed position after an injection operation, the protective assembly including a cover member (3;143;165;225) having an injection position relative to the body (11;161) in which the needle (13;162) is movable to the unsheathed position for effecting an injection and a post injection position relative to the body (11;161) in which the needle (13;162) is covered by the cover member (3;143;165;225), means for enabling relative movement between the cover member (3;143;165;225) and the body (11;161), and biasing means (4;145;166) for urging the cover member (3;143;165;225) to cover the needle (13;162) after the injection operation; **characterised in that** the cover member (3;143;165;225) has a flat front wall (21;146;168) extending from the sides of the cover member and in use transversely to the longitudinal axis of the injector (1;141;160), the front wall (21;146;168) being for locating against an injection surface and having a hole (22;169) through which the needle (13;162) can pass when the drive means (5,6) is released to drive the needle (13;162) from the sheathed position to the unsheathed position for effecting an injection with the cover member (3;143;165;225) in the injection position, and when the cover member (3;143;165;225) is urged to the post injection position after the injection operation to cover the needle (13;162) in the unsheathed position, and further means (152;155;167;170;225) to prevent the cover member (3;143;165;225) being retracted to the injection position to expose the needle (13;162) in the unsheathed position after the injection operation.

2. An injector as claimed in Claim 1 **characterised in that** the hole (22;169) aligns with an opening (164) for the needle (13;162) in the body (11;161) when the cover member (3;143;165;225) is in the injection position, and the opening is covered by a membrane (15) that is pierced by the needle (13;162) during the injection operation.

3. An injector as claimed in Claim 2 **characterised in that** the body (1') has a guide (18) with a bore defining the opening, and the bore has a conical portion (19) at an inner end and/or is covered by the membrane (15) at an outer end.

4. An injector as claimed in any preceding claim **characterised in that** the biasing means (166) urges the cover member (165) to a pre-injection position relative to the body (161) and the cover member (165;225) is movable from the pre-injection position to the injection position prior to release of the drive means (5,6).

5. An injector as claimed in Claim 4 **characterised in that** the longitudinal position of the cover member (165) relative to the body (161) is substantially the same in the pre-injection and post-injection positions.

6. An injector as claimed in Claim 4 **characterised in that** the longitudinal position of the cover member (225) relative to the body in the pre-injection position is between the post-injection and injection positions and is preferably about half, two-thirds or four-fifths of the way from the injection position towards the post injection position.

7. An injector as claimed in any one of Claims 4 to 6 **characterised in that** the biasing means (166) comprises a spring (166) that is compressed by movement of the cover member (165) from the pre-injection position to the injection position prior to the injection operation for automatically urging the cover member (165) to the post injection position after the injection operation.

8. An injector according to any one of Claims 4 to 7 **characterised in that** means is provided for releasably holding the cover member (225) in the pre-injection position.

9. An injector as claimed in any one of Claims 1 to 3 **characterised in that** means (23;26) is provided for releasably holding the cover member (3) in the injection position.

10. An injector as claimed in Claim 9 **characterised in that** the drive means (5,6) and the holding means (23;26) are releasable at substantially the same time to drive the needle (13) to the unsheathed position and free the biasing means (4) to urge the cover member (3) to the post injection position.

11. An injector as claimed in Claim 9 **characterised in that** the holding means (84;85) is releasable before the drive means (5,6) to free the biasing means (86) to urge the cover member (83) to a pre-injection position, and the cover member (83) is movable to the injection position against the biasing means (86) prior to releasing the drive means (5,6) and is urged by the biasing means (86) to the post-injection position after the injection operation.

12. An injector as claimed in any one of Claims 9 to 11 **characterised in that** the biasing means (166) comprises a spring (166) that is compressed in the injection position prior to the injection operation for automatically urging the cover member (165) to the post-injection position after the injection operation.

13. An injector as claimed in any preceding Claim **characterised in that** the cover member (3;143;165;225) is automatically locked on being urged to the post-injection position.

14. An injector as claimed in Claim 13 **characterised in that** the further means provided by the body (1') and, cover member (143;225) having co-operating formations (152;155) arranged to lock the cover member (143) in the post-injection position.

15. An injector as claimed in Claim 13 **characterised in that** the further means provided by the body (161) and cover member (165) being provided with misalignment means (167;170) to lock the cover member (165) in the post injection position.

16. An injector as claimed in any preceding claim **characterised in that** manually operable means (10;10') is provided for releasing the drive means (5,6).

17. An injector as claimed in Claim 16 **characterised in that** the biasing means (4;166) is weaker than the force required to release the drive means (5,6).

18. An injector as claimed in Claim 16 or Claim 17 **characterised in that** releasable means (9) is provided for rendering the injector (1;141;160;220) inoperable until released.

19. An injector as claimed in any preceding claim **characterised in that** the releasable drive means (5,6) includes a spring (5) held in a compressed state until the drive means (5,6) is released to free the spring to drive the needle (13;162) from the sheathed position to the unsheathed position.

20. An injector as claimed in Claim 19 **characterised in that** the spring (5) moves to a relatively decompressed state during the injection operation and cannot be re-compressed after the injection operation.

21. An injector as claimed in any preceding claim **characterised in that** the biasing means (166) urges the cover member (165;225) away from the body (161) to position the cover member (165;225) in a pre-injection position relative to the body (161) and the cover member (165;225) is movable from the pre-injection position to the injection position so as to compress the biasing means (166) prior to release of the drive means (5,6) whereby the biasing means (166) is operable to urge the cover member (165;225) from the injection position to the post injection position after the injection operation.

22. An injector as claimed in any preceding claim **characterised in that** the drive means (5,6) and the holding means (23;26) are releasable at substantially the same time at the beginning of the injection operation to drive the needle (13) to the unsheathed position and free the biasing means (4) to urge the cover member (3) to the post injection position, releasable means (9) for preventing actuation of the manually operable means (10) so as to render the injector (1) inoperable until release.

23. An injector as claimed in Claim 8 **characterised in that** the means for releasably holding the cover member (225) in the pre-injection position is a tear-off band.

24. An injector as claimed in Claim 9 **characterised in that** the means (23;26) for releasably holding the cover member (3) in the injection position is a latching element (23) disposed on the cover member (3) and co-operable with a latching element (26) disposed on the body (1').

25. An injector as claimed in Claim 14 **characterised in that** the co-operating formations (152;155) are arranged to lock the cover member (143;225) in the post-injection position by one-way snap engagement features (152;155;226).

26. An injector as claimed in Claim 15 **characterised in that** the misalignment means (167; 170) lock the cover member (165) in the post injection position by cocking, twisting or tilting the cover member (165) relative to the body (1') so that the needle (162) is misaligned with the aperture (169) in the cover member (165).

27. An injector as claimed in Claim 16 **characterised in that** the manually operable means (10;10') for releasing the drive means (5,6) is a graspable rear end cap (10;10') movable relative to the body (1) for releasing detent means (7,8) to drive the needle (13;162) from the sheathed position to the unsheathed position.

28. An injector as claimed in Claim 18 **characterised in that** releasable means (9) for rendering the injector (1) inoperable until released is a removable safety pin (9) preventing actuation of the manually operable means (10;10') until removed.

## Patentansprüche

1. Automatisches Injektionsgerät (1;141;160;220), umfassend ein Gehäuse (11;161), eine Medikamentenladung, welche in dem Gehäuse (11;161) enthalten ist, eine Nadel (13;162), welche anfänglich in einer eingeschlossenen Position innerhalb des Gehäuses (11;161) gehalten ist, eine auslösbare Antriebseinrichtung (5,6) zum Antreiben der Nadel (13;162) aus der eingeschlossenen Position in eine freigelegte Position, in welcher die Nadel (13;162) aus dem Gehäuse (11;161) vorsteht, wobei die auslösbare Antriebseinrichtung (5,6) ferner das Medikament durch die Nadel (13;162) drückt, eine Schutzeinrichtung zum Abdecken der Nadel (13;162) in der vorstehenden freiliegenden Position nach einem Injektionsvorgang, wobei die Schutzanordnung einen Deckelteil (3;143;165;225) umfasst, welcher eine Injektionsstellung bezüglich des Gehäuses (11;161) aufweist, in welcher die Nadel (13;162) in die ungeschützt vorstehende Position bewegbar ist, um eine Injektion durchzuführen und eine Stellung nach der Injektion bezüglich des Gehäuses (11;161), in welcher die Nadel (13;162) durch den Deckelteil (3;143;165;225) abgedeckt ist, Mittel zum Ermöglichen einer Relativbewegung zwischen dem Deckelteil (3;143;165;225) und dem Gehäuse (11;161), und Federmittel (4;145;166) zum Drücken des Deckelteils (3;143;165;225) zum Abdecken der Nadel (13;162) nach dem Injektionsvorgang, **dadurch gekennzeichnet, dass** der Deckelteil (3;143;165;225) eine flache Vorderwandung (21;146;168) aufweist, welche von den Seiten des Deckelteils ausgeht und bei der Benutzung quer zur Längsachse des Injektionsgeräts (1;141;160) liegt, wobei die Vorderwandung (21;146;168) zur Anordnung gegen eine Injektionsoberfläche vorgesehen ist und ein Loch (22;169) aufweist, durch welches die Nadel (13;162) hindurch laufen kann, wenn die Antriebseinrichtung (5,6) ausgelöst wird, um die Nadel (13;162) aus der eingeschlossenen Position in die freiliegende vorstehende Position anzutreiben, um eine Injektion mit dem Deckelteil (3;143;165;225) in der Injektionsposition durchzuführen, und wenn der Deckelteil (3;143;165;225) in die Nachinjektionsstellung nach der Position gedrückt wird, nachdem der Injektionsvorgang durchgeführt wurde, um die Nadel (13;162) in der vorstehenden freiliegenden Position abzudecken, und weitere Mittel (152,155;167,170;229), um zu verhindern, dass der Deckelteil (3;143;165;225) in die Injektionsposition zurückgezogen wird, um die Nadel (13;162) in der vorstehenden freiliegenden Position freizulegen, nachdem die Injektion durchgeführt wurde.

2. Injektionsgerät nach Anspruch 1, **dadurch gekennzeichnet, dass** das Loch (22;169) mit einer Öffnung (164) für die Nadel (13;162) in dem Gehäuse (11;161) fluchtet, wenn der Deckelteil (3;143;165;225) in der Injektionsposition befindlich ist, und dass die Öffnung durch eine Membran (15) abgedeckt ist, welche während des Injektionsvorganges durch die Nadel (13;162) perforiert wird.

3. Injektionsgerät nach Anspruch 2, **dadurch gekennzeichnet, dass** das Gehäuse (1') eine Führung (18) mit einer Bohrung, welche die Öffnung definiert, aufweist, und dass die Bohrung einen konischen Abschnitt (19) am Innenende aufweist und/oder durch die Membran (15) am Außenende abgedeckt ist.

4. Injektionsgerät nach einem der vorstehenden Ansprüche, **dadurch gekennzeichnet, dass** die Federmittel (166) den Deckelteil (165) in eine Vorinjektionsstellung bezüglich des Gehäuses (161) drücken, und dass der Deckelteil (165;225)) aus der Vorinjektionsstellung in die Injektionsstellung vor dem Auslösen der Antriebseinrichtung (5,6) bewegbar ist.

5. Injektionsgerät nach Anspruch 4, **dadurch gekennzeichnet, dass** die Position des Deckelteils (165) bezüglich des Gehäuses (161) in Längsrichtung im wesentlichen in der Vorinjektionsstellung die gleiche ist wie in den Nachinjektionsstellungen.

6. Injektionsgerät nach Anspruch 4, **dadurch gekennzeichnet, dass** die Position des Deckelteils (225) bezüglich des Gehäuses in Längsrichtung in der Vorinjektionsstellung zwischen der Nachinjektionsstellung und der Injektionsstellung liegt und bevorzugt bei der Hälfte, zwei Drittel oder vier Fünftel der Strecke von der Injektionsstellung in Richtung der Nachinjektionsstellung liegt.

7. Injektionsgerät nach einem der Ansprüche 5 bis 6, **dadurch gekennzeichnet, dass** das Federmittel (166) eine Feder (166) umfasst, welche durch die Bewegung des Deckelteils (165) aus der Vorinjektionsstellung in die Injektionsstellung vor dem Injektionsvorgang zusammengedrückt wird, um automatisch den Deckelteil in die Nachinjektionsstellung nach dem Injektionsvorgang zu drücken.

8. Injektionsgerät nach einem der Ansprüche 4 bis 7, **dadurch gekennzeichnet, dass** ein Mittel vorgesehen ist, um lösbar den Deckelteil (225) in der Vorinjektionsstellung zu halten.

9. Injektionsgerät nach einem der Ansprüche 1 bis 3, **dadurch gekennzeichnet, dass** Mittel (23;26) vorgesehen sind, um den Deckelteil (3) lösbar in der Injektionsstellung zu halten.

10. Injektionsgerät nach Anspruch 9, **dadurch gekennzeichnet, dass** die Antriebseinrichtung (5,6) und das Haltemittel (23;26) im wesentlichen gleichzeitig auslösbar sind, um die Nadel (13) in die vorstehende freiliegende Position anzutreiben und das Federmittel (4) freizusetzen, um den Deckelteil (3) in die Nachinjektionsstellung zu drücken.

11. Injektionsgerät nach Anspruch 9, **dadurch gekennzeichnet, dass** das Haltemittel (84;85) vor der Antriebseinrichtung (5,6) auslösbar ist, um das Federmittel (86) freizusetzen, um den Deckelteil (83) in eine Vorinjektionsstellung zu drücken, und dass der Deckelteil (83) in die Injektionsstellung gegen die Wirkung der Federmittel (86) bewegbar ist, ehe die Antriebseinrichtung (5,6) ausgelöst wird und durch das Federmittel (86) nach dem Injektionsvorgang in die Nachinjektionsstellung gedrückt wird.

12. Injektionsgerät nach einem der Ansprüche 9 bis 11, **dadurch gekennzeichnet, dass** das Federmittel (166) eine Feder (166) umfasst, welche in die Injektionsposition vor dem Injektionsvorgang zusammengedrückt wird, um selbsttätig den Deckelteil (165) nach dem Injektionsvorgang in die Nachinjektionsstellung zu drücken.

13. Injektionsgerät nach einem der vorstehenden Ansprüche, **dadurch gekennzeichnet, dass** der Deckelteil (3;143;165;225) nach dem Drücken in die Nachinjektionsstellung selbsttätig verriegelt wird.

14. Injektionsgerät nach Anspruch 13, **dadurch gekennzeichnet, dass** die weiteren, durch das Gehäuse (1') und den Deckelteil (143;225) bereitgestellten Mittel zusammenarbeitende Formationen (152;155) aufweisen, die derart ausgebildet sind, dass sie den Deckelteil (143) in der Nachinjektionsstellung verriegeln.

15. Injektionsgerät nach Anspruch 13, **dadurch gekennzeichnet, dass** das weitere, durch das Gehäuse (161) und den Deckelteil (165) bereitgestellte Mittel mit Versetzungsmitteln (167;170) versehen sind, um den Deckelteil (165) in der Nachinjektionsstellung zu verriegeln.

16. Injektionsgerät nach einem der vorstehenden Ansprüche, **dadurch gekennzeichnet, dass** ein manuell betätigbares Mittel (10;10') vorgesehen ist, um die Antriebseinrichtung (5,6) auszulösen.

17. Injektionsgerät nach Anspruch 16, **dadurch gekennzeichnet, dass** das Federmittel (4;166) schwächer ist als die zum Auslösen der Antriebseinrichtung (5,6) erforderliche Kraft.

18. Injektionsgerät nach Anspruch 16 oder Anspruch 17, **dadurch gekennzeichnet, dass** ein lösbares Mittel (9) vorgesehen ist, um das Injektionsgerät (1;141;160;220) bis zum Auslösen unbetätigbar zu machen.

19. Injektionsgerät nach einem der vorstehenden Ansprüche, **dadurch gekennzeichnet, dass** die auslösbare Antriebseinrichtung (5,6) eine Feder (5) einschließt, die in einem zusammengedrückten Zustand gehalten ist, bis die Antriebseinrichtung (5,6) ausgelöst wird, um die Feder zum Antreiben der Nadel (13;162) aus der eingeschtossfnen Position in die freiliegende vorstehende Position freizugeben.

20. Injektionsgerät nach Anspruch 19, **dadurch gekennzeichnet, dass** sich die Feder (5) in einen vergleichsweise entspannten Zustand während des Injektionsvorgangs bewegt, und nach dem Injektionsvorgang nicht wieder zusammengedrückt werden kann.

21. Injektionsgerät nach einem der vorstehenden Ansprüche, **dadurch gekennzeichnet, dass** das Federmittel (166) den Deckelteil (165;225) von dem Gehäuse (161) wegdrückt, um den Deckelteil (165;225) in eine Vorinjektionsstellung bezüglich des Gehäuses (161) zu bewegen, und dass der Deckelteil (165;225) aus der Vorinjektionsstellung in die Injektionsstellung bewegbar ist, um das Federmittel (166) vor dem Auslösen der Antriebseinrichtung (5,6) zusammenzudrücken, wodurch das Federmittel (166) betätigbar ist, um den Deckelteil (165;225) aus der Injektionsstellung in die Nachinjektionsstellung nach Durchführung des Injektionsvorgangs zu drücken.

22. Injektionsgerät nach einem der vorstehenden Ansprüche, **dadurch gekennzeichnet, dass** die Antriebseinrichtung (5,6) und die Halteeinrichtung (23;26) im wesentlichen gleichzeitig am Beginn des Injektionsvorgangs auslösbar sind, um die Nadel (13) in die vorstehend freiliegende Position anzutreiben und um das Federmittel (4) freizusetzen, um den Deckelteil (3) in die Nachinjektionsstellung zu drücken, mit einem lösbaren Mittel (9), um eine Betätigung des manuell betätigbaren Mittels zu verhindern, um **dadurch** das Injektionsgerät (1) bis zum Auslösen unbetätigbar zu machen.

23. Injektionsgerät nach Anspruch 8, **dadurch gekennzeichnet, dass** das Mittel zum lösbaren Halten des Deckelteils (225) in der Vorinjektionsstellung ein Abreißband ist.

24. Injektionsgerät nach Anspruch 9, **dadurch gekennzeichnet, dass** das Mittel (23;26) zum lösbaren Halten des Deckelteils (3) in der Injektionsstellung ein Riegelelement (23) ist, welches an dem Deckelteil (3) angeordnet ist und mit einem Riegelelement (26) zusammenarbeiten kann, welches an dem Gehäuse (1') angeordnet ist.

25. Injektionsgerät nach Anspruch 14, **dadurch gekennzeichnet, dass** die zusammenarbeitenden Formationen (152;155) derart angeordnet sind, dass sie den Deckelteil (225) in der Nachinjektionsstellung durch nur in einer Richtung wirksame Eingriffsmerkmale (152;155;226) verriegeln.

26. Injektionsgerät nach Anspruch 15, **dadurch gekennzeichnet, dass** die Versetzungsmittel (167;170) den Deckelteil (165) in der Nachinjektionsstellung durch Spannen, Verdrehen oder Neigen des Deckelteils (165) bezüglich des Gehäuses (1') verriegeln, so dass die Nadel (162) zur Öffnung (169) im Deckelteil (165) versetzt ist.

27. Injektionsgerät nach Anspruch 16, **dadurch gekennzeichnet, dass** das manuell betätigbare Mittel (10;10') zum Auslösen der Antriebseinrichtung (5,6) eine greifbare hintere Endkappe (10;10') ist, welche bezüglich des Gehäuses (1) beweglich ist, um ein Rastmittel (7,8) zum Antrieb der Nadel (13;162) aus der eingeschlossenen Position in die vorstehende freiliegende Position zu lösen.

28. Injektionsgerät nach Anspruch 18, **dadurch gekennzeichnet, dass** das lösbare Mittel (9) zum Unbetätigbarmachen des Injektionsgeräts (1) bis zum Auslösen ein herausnehmbarer Sicherheitsstift (9) ist, der eine Betätigung des manuell betätigbaren Mittels (10;10) verhindert, bis er entfernt wird.

## Revendications

1. Injecteur automatique (1; 141; 160; 220) comprenant un corps (1'; 161), une charge de médicament contenue dans le corps (1'; 161), une aiguille (13; 162) initialement maintenue dans une position gainée dans le corps (1'; 161), des moyens d'entrainement libérables (5; 6) pour entraîner l'aiguille (13; 162) depuis la position gainée vers une position non gainée dans laquelle l'aiguille (13; 162) dépasse du corps (1'; 161), les moyens d'entrainement libérables (5; 6) forçant également le médicament à travers de l'aiguille (13; 162), un assemblage de protection pour couvrir l'aiguille (13; 162) dans la position non gainée après une opération d'injection, l'assemblage de protection comprenant un élément couvercle (3; 143; 165; 225) ayant une position d'injection relativement au corps (1'; 161) dans laquelle l'aiguille (13; 162) est déplaçable vers la position non gainée pour effectuer une injection et une position de post-injection relativement au corps (1'; 161) dans laquelle l'aiguille (13; 162) est couverte par l'élément couvercle (3; 143; 165; 225), des moyens pour permettre le mouvement relatif entre l'élément couvercle (3; 143; 165; 225) et le corps (1'; 161), et des moyens de biaisement (4; 145; 166) pour pousser l'élément couvercle (3; 143; 165; 225) à couvrir l'aiguille (13; 162) après l'opération d'injection; **caractérisé en ce que** l'élément couvercle (3; 143; 165; 225) a une face frontale plate (21; 146; 168) s'étendant depuis les côtés de l'élément couvercle et en usage transversal à l'axe longitudinal de l'injecteur (1; 141; 160), la face frontale (212; 146; 168) étant pour localisation contre une surface d'injection et ayant un trou (22; 169) à travers lequel l'aiguille (13; 162) peut passer lorsque les moyens d'entrainement (5; 6) sont libérés pour entrainer l'aiguille (13; 162) depuis la position gainée vers la position non gainée pour effectuer une injection avec l'élément couvercle (3; 143; 165; 225) dans la position d'injection, et lorsque l'élément couvercle (3; 143; 165; 225) est poussé dans la position de post-injection après l'opération d'injection pour couvrir l'aiguille (13; 162) dans la position non gainée, et des moyens supplémentaires (152; 155; 167; 170 225) pour empêcher l'élément couvercle (3; 143; 165; 225) d'être rétracté dans la position d'injection pour exposer l'aiguille (13; 162) dans la position non gainée après l'opération d'injection.

2. Injecteur selon la revendication 1, **caractérisé en ce que** le trou (22; 169) est aligné avec une ouverture (164) pour l'aiguille (13; 162) dans le corps (1'; 161) lorsque l'élément couvercle (3; 143; 165; 225) est dans la position d'injection, et l'ouverture est couverte par une membrane (15) qui est percée par l'aiguille (13; 162) durant l'opération d'injection.

3. Injecteur selon la revendication 2, **caractérisé en ce que** le corps (1') a un guide (18) avec un perçage définissant l'ouverture, et le perçage à une portion conique (19) à une extrémité interne et/ou est couvert par une membrane (15) à une extrémité externe.

4. Injecteur selon l'une des revendications précédentes, **caractérisé en ce que** les moyens de biaisement (166) poussent l'élément couvercle (165) vers une position de pré-injection relativement au corps (161) et l'élément couvercle (165; 225) est déplaçable depuis la position de pré-injection vers la position d'injection avant la libération des moyens d'entrainement (5, 6).

5. Injecteur selon la revendication 4, **caractérisé en ce que** la position longitudinale de l'élément couvercle (165) relativement au corps (161) est sensiblement la même dans les positions de pré-injection et de post-injection.

6. Injecteur selon la revendication 4, **caractérisé en ce que** la position longitudinale de l'élément couvercle (225) relativement au corps dans la position de pré-injection est entre les positions de post-injection et d'injection et est de préférence à environ la moitié, les deux tiers ou les quatre cinquièmes du chemin depuis la position d'injection vers la position de post-injection.

7. Injecteur selon l'une quelconque des revendications 4 à 6, **caractérisé en ce que** les moyens de biaisement (166) comprennent un ressort qui est compressé par mouvement de l'élément couvercle (165) depuis la position de pré-injection vers la position d'injection avant l'opération d'injection pour automatiquement pousser l'élément couvercle (165) vers la position de post-injection après l'opération d'injection.

8. Injecteur selon l'une quelconque des revendications 4 à 7, **caractérisé en ce que** des moyens sont prévus pour maintenir de manière libérable l'élément couvercle (225) dans la position de pré-injection.

9. Injecteur selon l'une quelconque des revendications 1 à 3, **caractérisé en ce que** les moyens (23; 26) sont prévus pour maintenir de manière libérable l'élément couvercle (3) dans la position d'injection.

10. Injecteur selon la revendication 9, **caractérisé en ce que** les moyens d'entrainement (5; 6) et les moyens de maintien (23; 26) sont libérables sensiblement au même instant pour entrainer l'aiguille (13) vers la position non gainée et libérer des moyens de biaisement (4) pour pousser l'élément couvercle (3) vers la position de post-injection.

11. Injecteur selon la revendication 9, **caractérisé en ce que** les moyens de maintien (84; 85) sont libérables avant que les moyens d'entrainement (5; 6) ne libèrent les moyens de biaisement (86) pour pousser l'élément couvercle (83) vers une position de pré-injection, et l'élément couvercle (83) est déplaçable vers une position d'injection contre les moyens de biaisement (86) avant la libération des moyens d'entrainement (5; 6) et est poussé par les moyens de biaisement (86) vers la position de post-injection après l'opération d'injection.

12. Injecteur selon l'une quelconque des revendications 9 à 11, **caractérisé en ce que** les moyens de biaisement (166) comprennent un ressort (166) qui est comprimé dans la position d'injection avant l'opération d'injection pour automatiquement pousser l'élément couvercle (165) vers la position de post-injection après l'opération d'injection.

13. Injecteur selon l'une quelconque des revendications précédentes, **caractérisé en ce que** l'élément couvercle (3; 143; 165; 225) est automatiquement verrouillé en étant poussé vers la position de post-injection.

14. Injecteur selon la revendication 13, **caractérisé en ce que** les moyens supplémentaires prévus par le corps (1') et l'élément couvercle (143; 225) ont des formes de coopération (152; 155) arrangées pour verrouiller l'élément couvercle (143) dans la position de post-injection.

15. Injecteur selon la revendication 13, **caractérisé en ce que** les moyens supplémentaires prévus par le corps (161) et l'élément couvercle (165) sont prévus avec des moyens de désalignement (167; 170) pour verrouiller l'élément couvercle (165) dans la position de post-injection.

16. Injecteur selon l'une quelconque des revendications précédentes, **caractérisé en ce que** des moyens manuellement opérables (10; 10') sont prévus pour libérer les moyens d'entrainement (5;6).

17. Injecteur selon la revendication 16, **caractérisé en ce que** les moyens de biaisement (4; 166) sont plus faibles que la force requise pour libérer les moyens d'entrainement (5; 6).

18. Injecteur selon la revendication 16 ou 17, **caractérisé en ce que** les moyens libérables (9) sont prévus pour rendre l'injecteur (1; 141; 160; 220) inopérable jusqu'à libération.

19. Injecteur selon l'une des revendications précédentes, **caractérisé en ce que** les moyens d'entrainement libérables (5; 6) comprennent un ressort (5) maintenu dans un état comprimé jusqu'à ce que les moyens d'entrainement (5; 6) soient libérés pour libérer le ressort pour entrainer l'aiguille (13; 162) depuis la position gainée vers la position non gainée.

20. Injecteur selon la revendication 19, **caractérisé en ce que** le ressort (5) bouge vers un état relativement décompressé durant l'opération d'injection et ne puisse pas être re-compressé après l'opération d'injection.

21. Injecteur selon l'une des revendication précédentes, **caractérisé en ce que** les moyens de biaisement (166) poussent l'élément couvercle (165; 225) à l'extérieur du corps (161) pour positionner l'élément couvercle (165; 225) dans une position de pré-injection relativement au corps (161) et l'élément couvercle (165; 225) est déplaçable depuis la position de pré-injection vers la position d'injection pour comprimer les moyens de biaisement (166) avant de libérer les moyens d'entrainement (5; 6) par lesquels les moyens de biaisement (166) sont opérants pour pousser l'élément couvercle (165; 225) depuis la position d'injection vers la position de post-injection après l'opération d'injection.

22. Injecteur selon l'une des revendications précédentes **caractérisé en ce que** les moyens d'entrainement (5; 6) et les moyens de maintien (23; 26) sont libérables sensiblement au même instant au début de l'opération d'injection pour entrainer l'aiguille (13) vers la position non gainée et libérer les moyens de biaisement (4) pour pousser l'élément couvercle (3) vers la position de post-injection, des moyens libérables (9) pour prévenir l'actionnement des moyens opérables manuellement (10) pour rendre l'injecteur inopérant jusqu'à libération.

23. Injecteur selon la revendication 8, **caractérisé en ce que** les moyens pour maintenir de manière libérable l'élément couvercle (225) dans la position de pré-injection sont une bande détachable.

24. Injecteur selon la revendication 9, **caractérisé en ce que** les moyens (23; 26) pour maintenir de manière libérable l'élément couvercle (3) dans la position d'injection sont un élément à loquet (23) disposé sur l'élément couvercle (3) et co-opérable avec un élément à loquet (26) disposé sur le corps (1').

25. Injecteur selon la revendication 14, **caractérisé en ce que** les formes coopérantes (152; 155) sont arrangées pour verrouiller l'élément couvercle (143; 225) dans la position de post-injection par des accessoires à encliquetage unidirectionnel (152; 155; 226).

26. Injecteur selon la revendication 15, **caractérisé en ce que** les moyens de désalignement (167; 170) verrouillent l'élément couvercle (165) dans la position de post-injection en armant, en enroulant ou en inclinant l'élément couvercle (165) relativement au corps (1') pour que l'aiguille (162) soit désalignée de l'ouverture (169) dans l'élément couvercle (165).

27. Injecteur selon la revendication 16, **caractérisé en ce que** les moyens manuellement opérables (10; 10') pour libérer les moyens d'entrainement (5; 6) sont un capuchon (10, 10') empoignable sur l'extrémité arrière déplaçable relativement au corps (1) pour libérer des moyens d'encliquetage (7; 8) pour entraîner l'aiguille (13; 162) depuis la position gainée vers la position non gainée.

28. Injecteur selon la revendication 18, **caractérisé en ce que** les moyens libérables (9) pour rendre l'injecteur (1) inopérant jusqu'à libération sont une goupille de sécurité amovible (9) empêchant l'actionnement des moyens opérables manuellement (10; 10') jusqu'à ce qu'elle soit retirée.
